# EUROPEAN PATENT SPECIFICATION

(11) **EP 3 421 079 B1**
(45) Date of publication and mention of the grant of the patent: **19.01.2022**
(21) Application number: 17178941.5
(22) Date of filing: 30.06.2017
(51) Int. Cl.: A61N 1/05

(54) **LEAD FOR NEUROMODULATION**
LEITUNG FÜR NEUROMODULATION
CONDUCTEUR DE NEUROMODULATION

(43) Date of publication of application: 02.01.2019
(73) Proprietor: ONWARD Medical B.V., 5656 AE Eindhoven (NL)
(72) Inventor: GANTY, Damien, 5656 Eindhoven (NL); BAKKER, Bert, 5656 Eindhoven (NL); DELATTRE, Vincent, 5656 Eindhoven (NL); DECKERS, Sjaak, 5656 Eindhoven (NL)
(74) Representative: DTS Patent- und Rechtsanwälte Schnekenbühl und Partner mbB

(56) References cited:
- WO-A2-2013/142616
- US-A1- 2012 035 692
- US-A1- 2012 150 202
- US-A1- 2014 018 884
- US-A1- 2017 014 628
- US-B2- 9 044 592
- US-B2- 9 352 147

## Description

The present invention relates to a lead for neuromodulation and/or neurosensing, especially for neurostimulation and/or neurorecording, comprising at least one lead cable, at least one electrode section and at least one electrode placed in the electrode section.

Implantable leads with electrodes for spinal cord stimulation are known. In spinal cord stimulation, such electrodes are implanted into the spinal canal and more or less directly attached to the spinal cord. To provide the neurostimulation to the spinal cord in the planned manner, it is important to reach the correct implantation position and to fix the lead and the electrodes at this position.

It appears that existing anchoring techniques are not easy to establish in this context. In particular it has been reported that the lead might move when placing the anchor on the lead body. Alternatively, existing anchors do not allow fixing the lead properly or are not stable. Also, sliding of anchors along the lead body has been observed. Fixed anchors are already used for leads but such anchors do not form a convenient solution because of their shape size and specific location. Such fixed anchors may be established by small silicone parts, which are glued or overmolded on the lead body with wings and holes for a suture or the like.

It has also been reported that neurosurgeons in rare cases used the lead body for fixing sutures, thereby creating punctures in the lead body. Such punctures are, however, unwanted.

As an alternative to the above solutions, US 7,099,718 B1 relates to a neural stimulation lead fixation. The implantable lead has at least one electrode contact at or near its distal end prevents undesirable movement of the electrode contact from its initial implant location. One embodiment relates to a spinal cord stimulation (SCS) lead. A balloon may be positioned on the electrode lead array. The balloon is filled with air, liquid or a compliant material. When inflated, the balloon stabilizes the lead with respect to the spinal cord and holds the lead in place. The pressure of the balloon is monitored or otherwise controlled during the filling process in order to determine at what point the filling process should be discontinued. An elastic aspect of the balloon serves as a contained relief valve to limit the pressure the balloon may place on the surrounding tissues when the epidural space is constrained.

US2017014628A1 discloses a percutaneous stimulation lead adapted to be surgically implanted through a spinal needle or epidural needle. The lead is mechanically secured to a living tissue of a patient using either an anchor or a suture, passed through the tissue and then tied around the body of the lead. The anchor can be a simple sleeve or a locking anchor. The sleeve includes a central through hole through which the lead is passed. The locking anchor uses a set screw for locking purposes, and a bi-directional disposable torque wrench for locking and unlocking.

WO2013142616A2 discloses an electrical stimulation system including, inter alia, a lead formed by a paddle body and one or more lead bodies. A lead anchor can be used in an implantable device, such as an implantable spinal cord stimulator, to anchor a lead connecting a control module to an electrode array. The lead anchor includes a body and an exterior member, formed of a biocompatible material. The body contains at least one lead lumen through which a (respective) lead can pass. The interior of the lead lumen may be provided with concentric ridges or an interior thread for better engagement with the lead. The lead anchor includes a fastener, which is inserted into a transverse lumen and then tightened to hold the lead in place. The fastener can be a pin, clamp, latch, lug, nail, bolt, rod, rivet, screw or combinations thereof, or any other suitable item for engaging and anchoring the lead.

US2012150202A1 D3 discloses an implantable electrical stimulation lead including, inter alia, a suture sleeve including a sleeve member made of a biocompatible material. The suture sleeve may include a tubular body that defines at least one lumen extending through the length of the tubular body to receive at least a portion of a lead. The tubular body includes one or more flaps that can be used to secure the lead within the tubular body. When the one or more flaps are in a first position, the lead can readily be inserted through the lumen. Then, as the one or more flaps are switched to a second position, the first end of each flap contacts a portion of the lead disposed within the lumen of the tubular body to resist movement of the lead within the lumen, thereby securing the lead within the tubular body. One or more locking members can be provided to keep each flap in the second position. Alternatively, the suture sleeve can be made of, or otherwise include, an expandable material such that, as soon as the suture sleeve contacts a fluid, e.g., water, saline or blood, the expanding material expands so that the suture sleeve can be securely coupled to the lead inserted into the lumen. In some examples, the suture sleeve may include multiple suture sleeve base lumens in pre-set arrangements.

US2011178573A1 discloses a lead anchor including a fastener such as a screw for holding a lead in place within a lumen of the lead anchor.

US2008183257A1 discloses retention features, i.e., anchoring means, for anchoring or retaining leads, catheters or other devices in desired positions within the body of a patient. For instance, a retention feature may include a plurality of projections (e.g., having a hair-like or whisker-like shape extending radially outwardly from a lead body). Alternatively, the retention feature may comprise a coil wrapped around a section of the lead body having a reduced diameter. Still further, the retention feature may have a tubular structure, which can be positioned over the lead body and optionally advanced along the lead body for desired placement. Once the lead body is desirably placed, the retention feature is actuated to anchor the lead body in place.

US2012035692A1 discloses an implantable medical anchor for anchoring a lead, the anchor being capable of switching between a first, unlocked configuration and a second, locked configuration. The anchor includes a longitudinal body having a stationary member and a locking or moveable member, the movable member being capable of moving/rotating relative to the stationary member. In the first configuration, the movable member is unlocked and the lead can freely slide through a longitudinal lumen of the anchor. Then, when the anchor is switched to the second configuration, the movable member is locked with respect to the stationary member, so that the lumen is no longer linear and the flexible lead is forced to bend within the anchor. This bending substantially inhibits the lead from freely sliding through the longitudinal lumen and locks the anchor longitudinally in place relative to the lead.

US2014018884A1 discloses a lead anchor for securing at least one lead within a patient's body. The anchor includes an anchor housing provided with one or more (e.g., two) sleeves having respective channels through which a lead may pass. The channels may include a bend to provide a tapered form which may facilitate insertion of the leads. The anchor housing includes one or more locking members, e.g., pins, dowels, screws, bolts, or the like. When the anchor is in an unlocked configuration, the leads may move freely within the sleeves. Upon rotation of a swivel anchor, the anchor assumes an intermediate configuration where the swivel anchor compresses the sleeves and exerts pressure on the portions of the leads disposed in the sleeves. The intermediate configuration lies in an unstable equilibrium. Then, further rotation of the swivel anchor results in a stable, locked configuration where the leads are secured within the sleeves.

The invention is defined in the appended claims. Aspects, embodiments and examples disclosed herein which do not fall within the scope of the appended claims do not form part of the invention, and are merely provided for illustrative purposes.

It is therefore an object of the present invention to improve lead for neuromodulation and/or neurosensing, especially in that the lead can be fixed at the implantation site with high accuracy and that the fixation is easy to establish.

This object is solved according to the present invention by a lead for neuromodulation and/or neurosensing with the features of claim 1. Accordingly, a lead for neuromodulation and/or neurosensing is provided, especially a lead for neurostimulation and/or neurorecording, which comprises at least one lead cable, at least one electrode section and at least one electrode placed in the electrode section, wherein the lead comprises an anchoring section, which is pre-attached to the lead and configured to engage with tissue fixation means and/or hold a tissue fixation.

The lead may be a medical lead and it may be designed to be positioned for long-term implantation. The lead cable of the lead may be e.g. integral part and directly connected to the electrode section. The electrode section may be formed by one electrode at the tip end of the lead cable. Also, the electrode section may be also part of a paddle with a plurality of electrodes, together forming the electrode section.

The lead cable may be also detachable or a kind of extension cable.

Also, the lead cable may be formed just by a short connector being connected to the electrode section, wherein the connector is configured and arranged to be connected with an extension cable (e.g. for connection with another medical device and/or a controller and/or an implantable pulse generator (IPG)).

Specifically, the lead may be a lead for spinal cord neurostimulation and designed to be placed in the spinal canal, more specifically in the epidural or in the subdural space.

The tissue fixation with the tissue fixation means are selected from at least one of suture, suture fiber, clamp, bracket, staples or the like.

The anchoring means may be arranged in or within or close to the electrode section. For example, the anchoring means can be established by a hole within the paddle of the lead, such that e.g. sutures and/or clamps may be engaged with such holes. Also, it is possible that the anchoring means are positioned or arranged on or around at least one lead cable.

The invention is based on the basic idea that by pre-attaching at least one anchoring section to the lead, the position relative between lead and the anchoring means is already defined and set. So, no relative movement during surgery of the lead and the anchoring means is possible. Moreover, the position of the anchoring means can be used as additional guiding means for the surgeon, as the anchoring means may still be at least partially accessible or at least partially outside of the spinal canal and so be used as orientation means for placement of the lead. In particular the anchoring means may be arranged and configured such that they are capable to receive punctures for engaging and/or holding the fixation means. For example, the anchoring means may be flexible and/or elastic.

Moreover, it is possible that the anchoring section is a sleeve or sleeve-like section on the outer part of the lead cable, especially forming an outmost part of the lead cable.

The anchoring means are overmolded over and part of the outer part of the lead cable. By means of overmolding a simplified pre-attachment of the anchoring means to the lead can be provided. If the anchoring means is part of the outer part of the lead cable, it is possible to create such a section e.g. by varying the material thickness or by two or more component molding processes.

The anchoring means may be at least partially made of a thermoplastic material, especially a medical grade thermoplastic material, e.g. poly amide or poly ethylene polyurethane.

It is possible that the anchoring means may be at least partially made of an elastomeric material, especially a medical grade elastomeric thermoplastic material, e.g. medical grade silicone.

By this, punctures to the anchoring means for engaging with the fixation means like a suture or clamp or the like can be easily made and held by the anchoring means. At the same time, such material is at the one hand suitable to be implanted even for long-term applications and on the other hand mechanically stable enough to maintain the engagement with the fixation means over the life-time of the lead.

Furthermore, it is possible that the anchoring section has an uneven outer side. Such an uneven outer side may be formed by or with bumps, indentations or the like. Such bumps or indentations may be also used to keep wires and/or fiber of the suture in place, so that they cannot move away. Bumps can also be used to be targeted by and engaged with specifically clamps or the like.

The outer side of the anchoring section may be at least partially substantially pearly and/or comprises recesses and ridges. Such a structure can be easily formed e.g. by a molding process. Also, this structure can be used to keep wires and/or fiber of the suture in place, so that they cannot move away.

Additionally, it is possible that there are at least two anchoring means. So, more options for fixation may be provided to the surgeon. Also, both anchoring means may be used to enhance the fixation of the overall lead. It could, however, also envisioned that the fixation is sufficient with at least one engagement of one anchoring means with fixation means and that the one anchoring means is chosen by the surgeon, which is better accessible out the of the at least two anchoring means.

Moreover, in a further embodiment it is possible that the anchoring means close to the electrode section may be at a distal part of the lead cable. By such a layout the anchoring means is close to the electrode section and may therefore block and/or minimize unwanted movements and rotations of the electrode section. So, a correct positioning for the electrode section may be established. Also, this helps to keep the electrode positioning over a long time, e.g. over the complete lifetime of the implanted lead.

There may be an anchoring means at the proximal part of the lead cable. Such an anchoring means may be used to fix the lead cable in a position close to e.g. an implantable pulse generator or any other element that may be connected to the lead. This can be realized by providing a second anchoring means or by providing an elongated anchoring means, e.g. extending from a distal part of the lead cable to a proximal part of the lead cable.

Also, there may be between the anchoring means close to the electrode section and the electrode section a free lead cable section without anchoring section. This section may be used for freely forming and moving the lead cable in this part of the lead. So, the possibilities for steering and moving of the lead are enhanced.

Moreover, it is possible that the free lead cable section has a minimum length of chosen out of a range of approx. 5 mm, especially a length of chosen out of a range of approx. 10 mm to 60 mm. This range or ranges of the length for the free lead cable section is/are advantageous for enhancing the possibilities for steering and moving of the lead and at the same time having the anchoring section still close to the electrode section for providing a stable anchoring and fixation for/of the electrode section.

Additionally, it is possible that the anchoring section has a length of at least approx. 10 mm or more, especially approx. 60 mm or more.

Furthermore, the lead comprises a strain relief loop. Such a strain relief loop may be formed by a loop of one of the lead cables. It may be for example comprise less flexibility than other parts of the lead cable. The stiffening can be achieved and provided by the anchoring means.

The strain relief loop may be be pre-formed and/or pre-shaped.

This can be achieved by means of the anchoring means. In particular, the anchoring means may be stiff enough (but e.g. still formable) to form the strain relief loop. Then, it is possible that the anchoring means are pre-attached to the lead cable or more general to the lead cable, i.e. that the strain relief loop is already present when the lead is lying on a static position.

By means of the strain relief loop the application of strain and tension in the lead cable distal of the strain relief loop can be avoided or minimized. So, strain and tension are not forwarded to the electrode section, which helps keep the correct position of the electrodes.

The lead may be a spinal cord neurostimulation lead, especially wherein the electrode section is an electrode paddle with an array of electrodes, e.g. configured to be implanted in vicinity of the cervical or thoracic or lumbar or sacral section of the spinal cord.

It is generally also possible that the anchoring means is formed as part of the electrode section or as at least one hole in e.g. the electrode section of the lead.

The electrode section of the lead may comprise at least one anchoring means and/or may be directly connected to at least one anchoring means.

The anchoring means may be embodied as an extra lead part pre-attached to the electrode section.

The electrode section may be e.g. embodied as an electrode paddle.

The anchoring means may be not be attached to the lead cable but directly connected to the electrode section, e.g. the electrode paddle. The attachment of the anchoring means to the electrode section may be done by molding, overmolding or gluing or by forming a further integral part of the electrode section, e.g. by means of providing an extension or finger-like or wing-like or outgrowing part or paddle-like part, which serves as anchoring means.

The anchoring means may be made of semi-rigid materials (non-metallic except for the screw or the fixation means) such as a thermoplastic and/or elastomeric material and may be additionally or alternatively in a sleeve like or half sleeve form with wings for any suitable fixation means.

The anchoring means may have one or more hole to be attached with a suture fiber or a screw or a pin or clamp or any other suitable fixation means.

The anchoring means may not be linked (attached to) current-carrying parts such lead bodies or the lead cable in order to minimize applied constraint on electrical parts.

The present disclosure also relates to a method of fixing a lead for neuromodulation.

Accordingly, a method of fixing a lead for neuromodulation is provided, wherein the lead comprises at least one lead cable, at least one electrode section and at least one electrode placed in the electrode section, wherein the lead further comprises an anchoring section, the method comprising at least the following steps:
- the lead is placed at the implantation site,
- the lead is fixed at the implantation site by means of a fixation means being engaged with the surrounding tissue of the patient and engaged with the anchoring section.

The lead may be a lead as set forth above or described hereinafter.

Further details and advantages of the present invention shall now be disclosed in connection with the drawings.

It is shown in
- Fig. 1: a perspective view of a first embodiment of a lead according to the present invention;
- Fig. 2: a perspective view of a second embodiment of a lead according to the present invention;
- Fig. 3: a perspective view of a third embodiment of a lead according to the present invention;
- Fig. 4: a perspective view of a fourth embodiment of a lead according to the present invention;
- Fig. 5: a perspective view of a fifth embodiment of a lead according to the present invention;
- Fig. 6: a further view of a sixth embodiment of a lead according to the present invention;
- Fig. 7: a further view of the embodiment of Fig. 8 ;
- Fig. 8: a further view of a seventh embodiment of a lead according to the present invention; and
- Fig. 9: a flow chart of an example procedure of fixation of the lead.

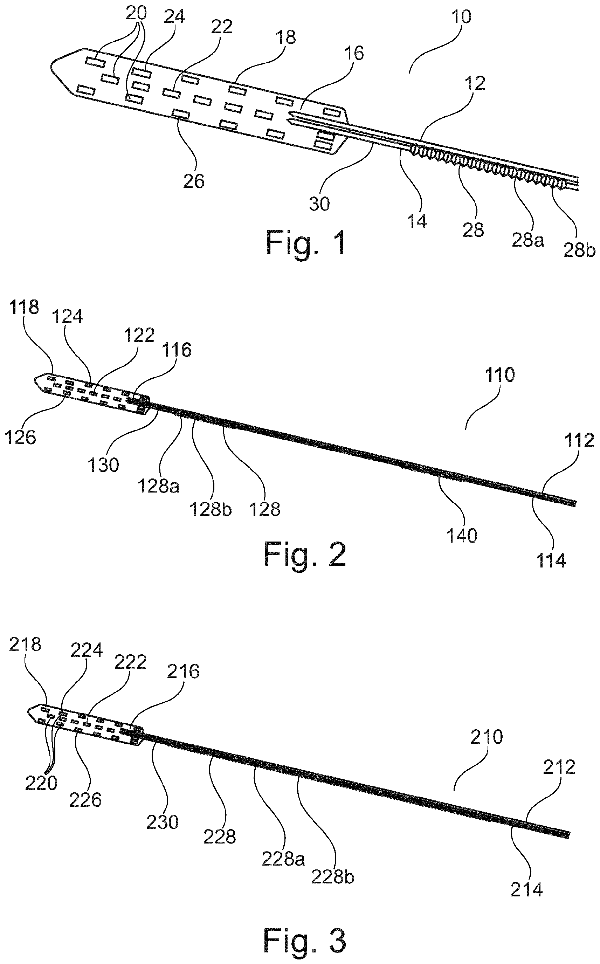

**Fig. 1** shows a perspective view of first embodiment of a lead 10 according to the present invention.

The lead 10 is a lead for neuromodulation and neurosensing, here for neurostimulation and neurorecording.

The lead 10 comprises two lead cables 12, 14 and an electrode section 16 arranged on an electrode paddle 18.

Specifically, the lead 10 is a spinal cord neurostimulation lead.

The electrode paddle 18 is made of flexible material, here comprising a silicone based material.

The electrode section 16 comprises three rows of electrodes 20, having an inner electrode row 22 in the middle of the electrode paddle 18 and two outer electrode rows 24, 26.

The electrode paddle 18 with the array of electrodes 20 is configured to be implanted in vicinity of the lumbar section of the spinal cord, especially to be placed in the spinal canal.

Generally speaking, such an electrode array or a similar electrode array may be used to be implanted in the cervical or thoracic or sacral section of the spinal cord

Furthermore, the lead 10 comprises an anchoring means 28, which is pre-attached to the lead 10 and configured to engage with tissue fixation means (not shown) and hold a tissue fixation.

The tissue fixation with the tissue fixation means are selected from at least one of suture, suture fiber, clamp, bracket, staples or the like.

The anchoring means 28 is here a sleeve-like section on the outer part of the lead cable 14, here forming an outermost part of the lead cable 14.

Moreover, the anchoring means 28 are overmolded over and part of the outer part of the lead cable 14.

The anchoring means 28 may at least partially be made of a thermoplastic material, especially a medical grade thermoplastic material, e.g. poly amide or poly ethylene polyurethane.

However, here the anchoring means 28 is at least partially made of an elastomeric material, namely a medical grade elastomeric thermoplastic material, here medical grade silicone.

Furthermore, the anchoring means 28 has a length 20. Normally, it can have a length of approx. 100 mm or more, especially approx. 60 mm or more.

As can be seen in Fig. 1, the anchoring means 28 comprises an uneven outer side, i.e. it is pearly and comprises recesses 28a and ridges 28b. Also, it can be described as having a plurality of bumps, here identically shaped bumps 28b.

Between the anchoring means 28 close to the electrode section 16 and the electrode paddle 18 and the electrode section is a free lead cable section 30 without anchoring means.

The free lead cable section 30 has a length of approx. 20 mm. Normally, the free lead cable section has a minimum length of chosen out of a range of approx. 5 mm, especially a length of chosen out of a range of approx. 10 mm to 60 mm.

**Fig. 2** shows a perspective view of a second embodiment of a lead 110 according to the present invention.

The lead 110 comprises each and every structural and functional feature as shown in Fig. 1 and as described above.

Similar or identical features have the same reference number, however increased by the value 100.

As an additional feature, the lead 110 comprises a second anchoring means 140.

The second anchoring section 140 is spaced apart from the first anchoring means 128 on the same lead cable 114.

The second anchoring means 140 is at the proximal part of the lead cable 114, so that the lead cable 114 may be anchored in a section close to the connection to an implantable pulse generator (IPG).

**Fig. 3** shows a perspective view of a third embodiment of a lead 210 according to the present invention.

The lead 210 comprises each and every structural and functional feature as shown in Fig. 1 and as described above.

Similar or identical features have the same reference number, however increased by the value 200.

As an additional feature, the lead 110 comprises an elongated anchoring means 228, which covers more than 2/3 of the length of the lead cable 214. So, the same anchoring means 228 may be used for a fixation close to the proximal part and the distal part of the lead cable 214.

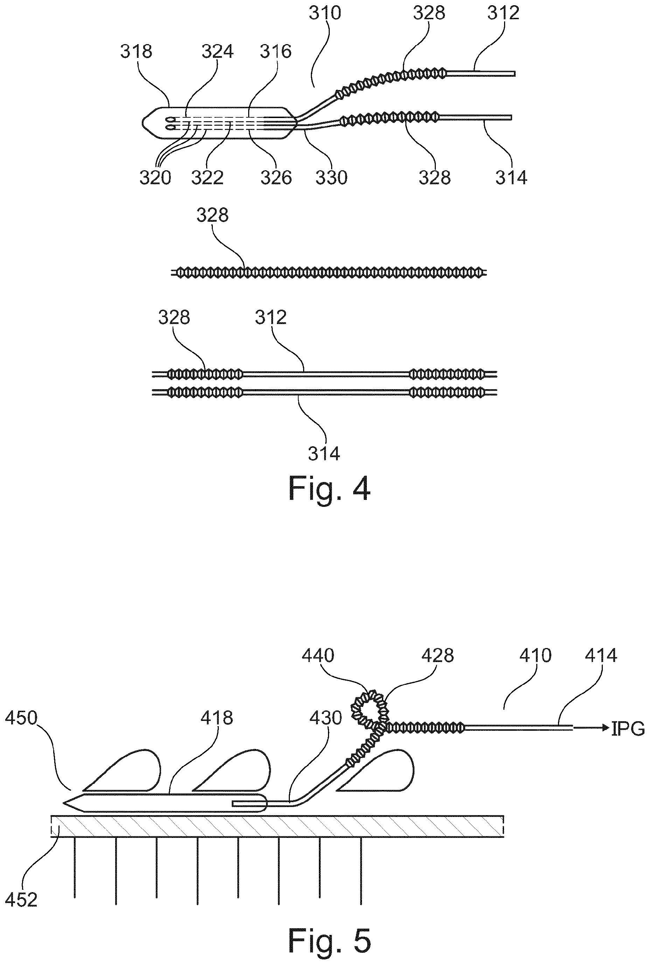

**Fig. 4** shows a perspective view of a fourth embodiment of a lead 310 according to the present invention.

The lead 310 comprises each and every structural and functional feature as shown in Fig. 1 and as described above.

Similar or identical features have the same reference number, however increased by the value 300.

As an additional feature, the lead 310 comprises anchoring means 328 and 328a at each lead cable in the distal area of lead cables 312 and 314.

It is also possible, that there is on both lead cable a second proximal anchoring means likewise the second anchoring means 140.

**Fig. 5** shows a perspective view of a fifth embodiment of a lead 410 according to the present invention.

The lead 410 comprises each and every structural and functional feature as shown in Fig. 1 and as described above.

Similar or identical features have the same reference number, however increased by the value 400.

As an additional feature, the lead 410 comprises a strain relief loop 440.

The whole strain relief loop 440 is covered with the anchoring means 428.

The function of the strain relief loop is to allow application of strain and tension in the lead cable 414 distal of the strain relief loop 440, so that strain and tension are not forwarded to the electrode paddle 418.

The electrode paddle 418 is here shown in Fig. 5 as being placed in the spinal canal 450 close to the lumbar spinal cord 452.

As can be also seen, the strain relief loop 440 is outside of the spinal canal 450 and also outside of the vertebrae. So, a fixation can be done with the tissue surrounding the vertebrae at this location.

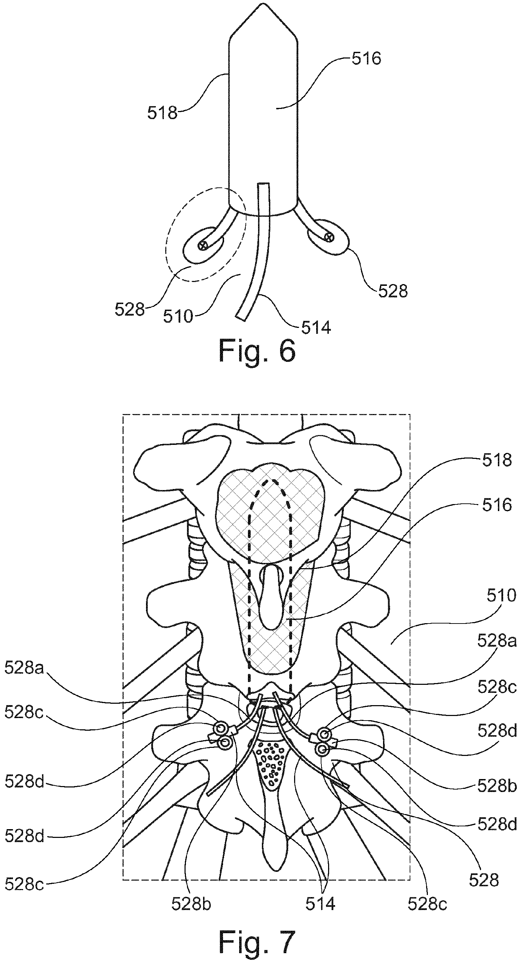

**Fig. 6** and **Fig. 7** show a sixth embodiment of a lead 510 according to the present invention.

Fig. 7 shows the implanted lead in the implantation site of a human vertebrae and spinal cord at the lumbar part of the spinal cord.

The lead 510 comprises each and every structural and functional feature as shown in Fig. 1 and as described above.

Similar or identical features have the same reference number, however increased by the value 500.

As additional features, the lead 510 comprises instead of anchoring means attached to the lead cable(s) two paddle anchoring means 528, which are directly attached and connected to the electrode section 516, here being formed on and arranged on the electrode paddle 518.

The two paddle anchoring means 528 are arranged here on both sides of the lead cable 514 or lead cables 514 (as shown in Fig. 7) at the proximal end or proximal part of the electrode paddle 518.

Moreover, the paddle anchoring means 528 is not attached to the lead cables 518 but only and directly connected to the electrode paddle 518.

So, the paddle anchoring means 528 are not be linked (attached to) current-carrying parts such lead bodies or the lead cable in order to minimize applied constraint on electrical parts.

The attachment of the anchoring means to the electrode section may be done by molding, overmolding or gluing or by forming a further integral part of the electrode section, e.g. by means of providing an extension or finger-like or wing-like or outgrowing part or paddle-like part, which serves as anchoring means.

Here, they are formed sleeve-like on a paddle-extension cable 528a as having a sleeve 528b with two wings 528c.

In each wing 528c there is a hole 528d to be attached with a suture fiber or a screw or a pin or clamp or any other suitable fixation means.

The paddle anchoring means 528 may be made of semi-rigid materials (non-metallic except for the screw or the fixation means) such as a thermoplastic and/or elastomeric material and may be additionally or alternatively in a sleeve like or half sleeve form with wings for any suitable fixation means.

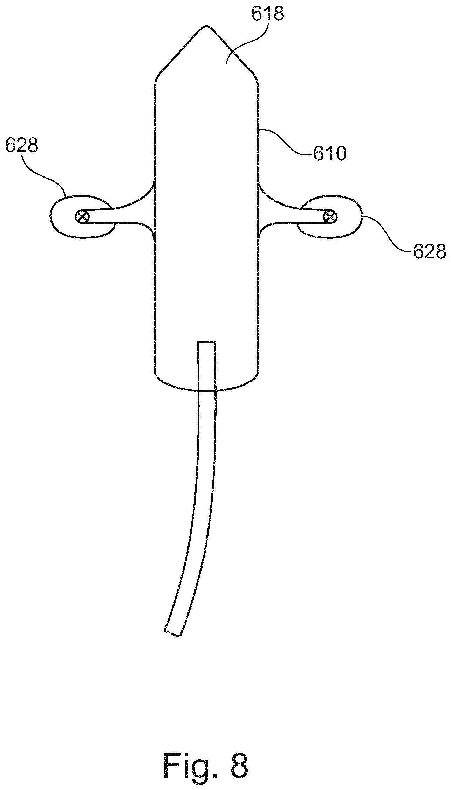

Fig. 8 shows a seventh embodiment of a lead according to the present invention.

The lead 610 comprises each and every structural and functional feature as shown in Fig. 6 and Fig. 7 and as described above.

Similar or identical features have the same reference number, however increased by the value 100 compared to the reference number of Fig. 6 and Fig. 7.

The only difference is the fact that the paddle anchoring means 628 are arranged in a middle section at the sides of the electrode paddle 618.

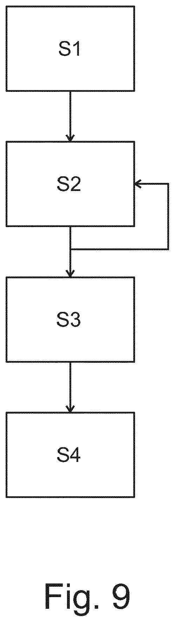

Fig. 9 shows a flow chart of an example procedure of fixation of the lead, here the lead 10.

The fixation of the lead 10 comprises the following steps:
Firstly, in step S1, the lead 10 is placed at the implantation site by the surgeon. Here, the surgeon positions the lead 10 with the electrode paddle 18 in the spinal canal, e.g. in vicinity of the lumbar section of the spinal cord.

In the second step S2 the electrodes 22, 24, 26 and thus the placement of the electrode section 16 is controlled, e.g. by several testings. For example, test stimulation patterns can be used and the response thereto checked. There may be iterations of this step. If the test confirms correct placement, then it is continued with third step S3.

By performing some tests in step S2 the correct and intended placement and position of the lead 10 with the electrode paddle 18 is confirmed.

Then, in the third step S3, the lead 10 is fixed at the implantation site by means of a fixation means being engaged with the surrounding tissue of the patient and engaged with the anchoring means 28.

For example, this can be done by engaging a suture with the anchoring means 28 and surrounding tissue, as mentioned in step S4. The suture fiber can then be wound around the bumps 28b and is held in the recesses 28a.

In the fourth step S4 then the surgery is completed, i.e. by continuing with the implantation of other elements of an implantable medical system and/or finalization of the surgical intervention.

### References

- 10: lead
- 12: lead cable
- 14: lead cable
- 16: electrode section
- 18: electrode paddle
- 20: electrode
- 22: inner electrode row
- 24: outer electrode row
- 26: outer electrode row
- 28: anchoring means
- 28a: recess
- 28b: ridge, bump
- 30: free lead cable section

- 110: lead
- 112: lead cable
- 114: lead cable
- 116: electrode section
- 118: electrode paddle
- 120: electrode
- 122: inner electrode row
- 124: outer electrode row
- 126: outer electrode row
- 128: anchoring means
- 128a: recess
- 128b: ridge, bump
- 130: free lead cable section
- 140: second anchoring means

- 210: lead
- 212: lead cable
- 214: lead cable
- 216: electrode section
- 218: electrode paddle
- 220: electrode
- 222: inner electrode row
- 224: outer electrode row
- 226: outer electrode row
- 228: anchoring means
- 228a: recess
- 228b: ridge, bump
- 230: free lead cable section

- 310: lead
- 312: lead cable
- 314: lead cable
- 316: electrode section
- 318: electrode paddle
- 320: electrode
- 322: inner electrode row
- 324: outer electrode row
- 326: outer electrode row
- 328: anchoring means
- 328a: recess
- 328b: ridge, bump
- 330: free lead cable section

- 410: lead
- 412: lead cable
- 414: lead cable
- 416: electrode section
- 418: electrode paddle
- 420: electrode
- 422: inner electrode row
- 424: outer electrode row
- 426: outer electrode row
- 428: anchoring means
- 428a: recess
- 428b: ridge, bump
- 430: free lead cable section
- 440: strain relief loop

- 510: lead
- 514: lead cable
- 516: electrode section
- 518: electrode paddle
- 528: anchoring means
- 528a: paddle-extension cable
- 528b: sleeve
- 528c: wing
- 528d: hole

- S1: step 1 of fixation method
- S2: step 2 of fixation method
- S3: step 3 of fixation method
- S4: step 4 of fixation method

## Claims

1. A lead (10; 110; 210; 310; 410) for neuromodulation and/or neurosensing, especially for neurostimulation and/or neurorecording, comprising at least one lead cable (12, 14; 112, 114; 212, 214; 312, 314; 412, 414), at least one electrode section (16; 116; 216; 316; 416) and at least one electrode (20; 120; 220; 320; 420) placed in the electrode section (16; 116; 216; 316; 416), wherein the lead (10; 110; 210; 310; 410) comprises an anchoring means (28; 128; 228; 328; 428), which are pre-attached to the lead (10; 110; 210; 310; 410), wherein the anchoring means (28; 128; 228; 328; 428) is a sleeve or sleeve-like section on the outer part of the lead cable (14; 114; 214; 314; 414), especially forming an outermost part of the lead cable (14; 114; 214; 314; 414) wherein, by means of the pre-attaching of the anchoring means (28; 128; 228; 328; 428) the position relative between lead and anchoring means is defined and set such that no relative movement of the lead and anchoring means is possible,
**characterized in that** the anchoring means (28; 128; 228; 328; 428) is overmolded over and
part of the outer part of the lead cable (14; 114; 214; 314; 414).

2. The lead (10; 110; 210; 310; 410) according to claim 1, **characterized in that** the anchoring means (28; 128; 228; 328; 410) is at least partially made of a thermoplastic material, especially a medical grade thermoplastic material, e.g., polyamide or polyethylene polyurethane.

3. The lead (10; 110; 210; 310; 410) according to one of the preceding claims, **characterized in that** the anchoring means (28; 128; 228; 328; 428) is at least partially made of an elastomeric material, especially a medical grade elastomeric thermoplastic material, e.g., medical grade silicone.

4. The lead (10; 110; 210; 310; 410) according to one of the preceding claims, **characterized in that** the anchoring means (28; 128; 228; 328; 428) has an uneven outer side.

5. The lead (10; 110; 210; 310; 410) according to claim 4, **characterized in that** the outer side of the anchoring means (28; 128; 228; 328; 428) is at least partially pearly and/or comprises recesses (28a; 128a; 228a; 328a; 428a) and ridges (28b; 128b; 228b; 328b; 428b).

6. The lead (110; 310) according to one of the preceding claims, **characterized in that** there are at least two anchoring means (128, 140; 328, 328a).

7. The lead according (10; 110; 210; 310; 410) to one of the preceding claims, **characterized in that** the anchoring means (28; 128; 228; 328; 428) close to the electrode section is at a distal part of the lead cable (14; 114; 214; 314; 414).

8. The lead (110; 210; 310; 410) according to one of the preceding claims, **characterized in that** there is an anchoring means (140; 228; 328; 428) at the proximal part of the lead cable (114; 214; 314; 414).

9. The lead (10; 110; 210; 310; 410) according to claim 6 or claim 7, **characterized in that** between the anchoring means (28; 128; 228; 328; 428) close to the electrode section (16; 116; 216; 316; 416) and the electrode section (16; 116; 216; 316; 416) is a free lead cable section (30; 130; 230; 330; 430) without anchoring means.

10. The lead (10; 110; 210; 310; 410) according to claim 9, **characterized in that** the free lead cable section (30; 130; 230; 330; 430) has a minimum length of chosen out of a range of 5 mm, especially a length of chosen out of a range of 10 mm to 60 mm and/or that the anchoring means (28; 128; 228; 328; 428) has a length of at least 10 mm or more, especially 60 mm or more.

11. The lead (410) according to one of the preceding claims, **characterized in that** the lead (410) comprises a strain relief loop (440).

12. The lead (10; 110; 210; 310; 410) according to one of the preceding claims, **characterized in that** the lead (10; 110; 210; 310; 410) is a spinal cord neurostimulation lead, especially wherein the electrode section (16; 116; 216; 316; 416) is an electrode paddle (18; 118; 218; 318; 418) with an array of electrodes (20; 120; 220; 320; 420), e.g., configured to be implanted in vicinity of the cervical or thoracic or lumbar or sacral section of the spinal cord.

13. The lead (510; 610) according to one of the preceding claims, **characterized in that** the electrode section (516; 616) of the lead (510; 610) comprises at least one anchoring means (528; 628) and/or is directly connected to at least one anchoring means (528; 628).

14. The lead (110) according to claim 1, **characterized in that** it further comprises a second anchoring means (140), spaced apart from the anchoring means (128) on the same lead cable (114).

15. The lead (110) according to claim 14, **characterized in that** the second anchoring means (140) is formed at the proximal part of the lead cable (114).

16. The lead (210) according to claim 1, **characterized in that** the anchoring means (228) is configured to cover more than 2/3 of the length of the lead cable (210).

17. The lead (310) according to claim 1, wherein the lead includes a plurality of lead cables (312, 314), **characterized in that** the anchoring means (328, 328a) is provided at the distal end of each of the plurality of lead cables (312, 314), optionally wherein a second anchoring means is provided at the proximal end of each of the plurality of lead cables (312, 314).

18. The lead (410) according to claim 11, **characterized in that** the strain relief loop (440) is covered with the anchoring means (428).

## Patentansprüche

1. Leitung (10; 110; 210; 310; 410) zur Neuromodulation und/oder Neuroerfassung, insbesondere zur Neurostimulation und/oder Neuroaufzeichnung, mit mindestens einem Leitungskabel (12, 14; 112, 114; 212, 214; 312, 314; 412, 414), mindestens einem Elektrodenabschnitt (16; 116; 216; 316; 416) und mindestens einer im Elektrodenabschnitt (16; 116; 216; 316; 416) platzierten Elektrode (20; 120; 220; 320; 420), wobei die Leitung (10; 110; 210; 310; 410) eine Verankerungseinrichtung (28; 128; 228; 328; 428) aufweist, die an der Leitung (10; 110; 210; 310; 410) vorab angebracht ist, wobei es sich bei der Verankerungseinrichtung (28; 128; 228; 328; 428) um eine Hülse oder einen hülsenartigen Abschnitt am Außenteil des Leitungskabels (14; 114; 214; 314; 414) handelt, wobei sie insbesondere einen äußersten Teil des Leitungskabels (14; 114; 214; 314; 414) bildet, wobei durch die vorab erfolgende Anbringung der Verankerungseinrichtung (28; 128; 228; 328; 428) die Relativposition zwischen Leitung und Verankerungseinrichtung so definiert und eingestellt ist, dass keine Relativbewegung von Leitung und Verankerungseinrichtung möglich ist,
**dadurch gekennzeichnet, dass** die Verankerungseinrichtung (28; 128; 228; 328; 428) an den Außenteil des Leitungskabels (14; 114; 214; 314; 414) angeformt ist und einen Teil davon darstellt.

2. Leitung (10; 110; 210; 310; 410) nach Anspruch 1, **dadurch gekennzeichnet, dass** die Verankerungseinrichtung (28; 128; 228; 328; 410) mindestens teilweise aus einem thermoplastischen Material besteht, insbesondere aus einem thermoplastischen Material medizinischer Qualität, z. B. Polyamid oder Polyethylen-Polyurethan.

3. Leitung (10; 110; 210; 310; 410) nach einem der vorhergehenden Ansprüche, **dadurch gekennzeichnet, dass** die Verankerungseinrichtung (28; 128; 228; 328; 428) mindestens teilweise aus einem Elastomermaterial besteht, insbesondere einem thermoplastischen Elastomermaterial medizinischer Qualität, z. B. medizinischem Silikon.

4. Leitung (10; 110; 210; 310; 410) nach einem der vorhergehenden Ansprüche, **dadurch gekennzeichnet, dass** die Verankerungseinrichtung (28; 128; 228; 328; 428) eine unebene Außenseite hat.

5. Leitung (10; 110; 210; 310; 410) nach Anspruch 4, **dadurch gekennzeichnet, dass** die Außenseite der Verankerungseinrichtung (28; 128; 228; 328; 428) zumindest teilweise perlenartig ist und/oder Vertiefungen (28a; 128a; 228a; 328a; 428a) und Stege (28b; 128b; 228b; 328b; 428b) aufweist.

6. Leitung (110; 310) nach einem der vorhergehenden Ansprüche, **dadurch gekennzeichnet, dass** mindestens zwei Verankerungseinrichtungen (128, 140; 328, 328a) vorhanden sind.

7. Leitung (10; 110; 210; 310; 410) nach einem der vorhergehenden Ansprüche, **dadurch gekennzeichnet, dass** sich die Verankerungseinrichtung (28; 128; 228; 328; 428) nahe am Elektrodenabschnitt an einem distalen Teil des Leitungskabels (14; 114; 214; 314; 414) befindet.

8. Leitung (110; 210; 310; 410) nach einem der vorhergehenden Ansprüche, **dadurch gekennzeichnet, dass** sich eine Verankerungseinrichtung (140; 228; 328; 428) am proximalen Teil des Leitungskabels (114; 214; 314; 414) befindet.

9. Leitung (10; 110; 210; 310; 410) nach Anspruch 6 oder Anspruch 7, **dadurch gekennzeichnet, dass** sich zwischen der Verankerungseinrichtung (28; 128; 228; 328; 428) nahe am Elektrodenabschnitt (16; 116; 216; 316; 416) und dem Elektrodenabschnitt (16; 116; 216; 316; 416) ein freier Leitungskabelabschnitt (30; 130; 230; 330; 430) ohne Verankerungseinrichtung befindet.

10. Leitung (10; 110; 210; 310; 410) nach Anspruch 9, **dadurch gekennzeichnet, dass** der freie Leitungskabelabschnitt (30; 130; 230; 330; 430) eine Mindestlänge aufweist, die aus einem Bereich von 5 mm ausgewählt ist, insbesondere eine Länge, die aus einem Bereich von 10 mm bis 60 mm ausgewählt ist, und/oder dass die Verankerungseinrichtung (28; 128; 228; 328; 428) eine Länge von mindestens 10 mm oder mehr, insbesondere 60 mm oder mehr hat.

11. Leitung (410) nach einem der vorhergehenden Ansprüche, **dadurch gekennzeichnet, dass** die Leitung (410) eine Zugentlastungsschlaufe (440) aufweist.

12. Leitung (10; 110; 210; 310; 410) nach einem der vorhergehenden Ansprüche, **dadurch gekennzeichnet, dass** die Leitung (10; 110; 210; 310; 410) eine Rückenmark-Neurostimulationsleitung ist, insbesondere wobei es sich bei dem Elektrodenabschnitt (16; 116; 216; 316; 416) um eine Elektrodenlamelle (18; 118; 218; 318; 418) mit einer Anordnung aus Elektroden (20; 120; 220; 320; 420), handelt, die z.B. dafür ausgelegt ist, im Nahbereich des zervikalen oder thorakalen oder lumbalen oder sakralen Abschnitt des Rückenmarks implantiert zu werden.

13. Leitung (510; 610) nach einem der vorhergehenden Ansprüche, **dadurch gekennzeichnet, dass** der Elektrodenabschnitt (516; 616) der Leitung (510; 610) mindestens eine Verankerungseinrichtung (528; 628) aufweist und/oder mit mindestens einer Verankerungseinrichtung (528; 628) direkt verbunden ist.

14. Leitung (110) nach Anspruch 1, **dadurch gekennzeichnet, dass** sie darüber hinaus eine zweite Verankerungseinrichtung (140) aufweist, die von der Verankerungseinrichtung (128) an demselben Leitungskabel (114) beabstandet ist.

15. Leitung (110) nach Anspruch 14, **dadurch gekennzeichnet, dass** die zweite Verankerungseinrichtung (140) am proximalen Teil des Leitungskabels (114) ausgebildet ist.

16. Leitung (210) nach Anspruch 1, **dadurch gekennzeichnet, dass** die Verankerungseinrichtung (228) so ausgelegt ist, dass sie mehr als 2/3 der Länge des Leitungskabels (210) bedeckt.

17. Leitung (310) nach Anspruch 1, wobei die Leitung mehrere Leitungskabel (312, 314) umfasst, **dadurch gekennzeichnet, dass** die Verankerungseinrichtung (328, 328a) am distalen Ende von jedem der mehreren Leitungskabel (312, 314) vorgesehen ist, optional wobei eine zweite Verankerungseinrichtung am proximalen Ende von jedem der mehreren Leitungskabel (312, 314) vorgesehen ist.

18. Leitung (410) nach Anspruch 11, **dadurch gekennzeichnet, dass** die Zugentlastungsschlaufe (440) von der Verankerungseinrichtung (428) bedeckt ist.

## Revendications

1. Conducteur (10 ; 110 ; 210 ; 310 ; 410) de neuromodulation et/ou de neurodétection, particulièrement de neurostimulation et/ou de neuroenregistrement, comprenant au moins un câble conducteur (12, 14 ; 112, 114 ; 212, 214 ; 312, 314 ; 412, 414), au moins une section d'électrode (16 ; 116 ; 216 ; 316 ; 416) et au moins une électrode (20 ; 120 ; 220 ; 320 ; 420) placée dans la section d'électrode (16 ; 116 ; 216 ; 316 ; 416), dans lequel le conducteur (10 ; 110 ; 210 ; 310 ; 410) comprend un moyen d'ancrage (28 ; 128 ; 228 ; 328 ; 428) qui est préfixé au conducteur (10 ; 110 ; 210 ; 310 ; 410), dans lequel le moyen d'ancrage (28 ; 128 ; 228 ; 328 ; 428) est un manchon ou une section similaire à un manchon placé sur la partie extérieure du câble conducteur (14 ; 114 ; 214 ; 314 ; 414), formant particulièrement une partie la plus à l'extérieur du câble conducteur (14 ; 114 ; 214 ; 314 ; 414) dans lequel la position relative entre le conducteur et le moyen d'ancrage est définie et paramétrée de telle sorte, au moyen de la préfixation du moyen d'ancrage (28 ; 128 ; 228 ; 328 ; 428), qu'aucun mouvement relatif du conducteur et du moyen d'ancrage n'est possible ; **caractérisé en ce que** le moyen d'ancrage (28 ; 128 ; 228 ; 328 ; 428) est surmoulé sur la partie extérieure du câble conducteur (14 ; 114 ; 214 ; 314 ; 414) et fait partie d'elle.

2. Conducteur (10 ; 110 ; 210 ; 310 ; 410) selon la revendication 1, **caractérisé en ce que** le moyen d'ancrage (28 ; 128 ; 228 ; 328 ; 410) est au moins en partie fabriqué à partir d'un matériau thermoplastique, particulièrement un matériau thermoplastique à grade médical, par exemple du polyamide ou du polyéthylène ou du polyuréthane.

3. Conducteur (10 ; 110 ; 210 ; 310 ; 410) selon l'une quelconque des revendications précédentes, **caractérisé en ce que** le moyen d'ancrage (28 ; 128 ; 228 ; 328 ; 428) est au moins en partie fabriqué à partir d'un matériau élastomère, particulièrement un matériau thermoplastique élastomère à grade médical, par exemple un silicone à grade médical.

4. Conducteur (10 ; 110 ; 210 ; 310 ; 410) selon l'une quelconque des revendications précédentes, **caractérisé en ce que** le moyen d'ancrage (28 ; 128 ; 228 ; 328 ; 428) a un côté extérieur irrégulier.

5. Conducteur (10 ; 110 ; 210 ; 310 ; 410) selon la revendication 4, **caractérisé en ce que** le côté extérieur du moyen d'ancrage (28 ; 128 ; 228 ; 328 ; 428) est au moins en partie nacré et/ou comprend des renfoncements (28a ; 128a ; 228a ; 328a ; 428a) et des bombements (28b ; 128b ; 228b ; 328b ; 428b).

6. Conducteur (110 ; 310) selon l'une quelconque des revendications précédentes, **caractérisé en ce qu'**il y a au moins deux moyens d'ancrage (128, 140 ; 328, 328a).

7. Conducteur (10 ; 110 ; 210 ; 310 ; 410) selon l'une quelconque des revendications précédentes, **caractérisé en ce que** le moyen d'ancrage (28 ; 128 ; 228 ; 328 ; 428) situé à proximité de la section d'électrode se place au niveau d'une partie distale du câble conducteur (14 ; 114 ; 214 ; 314 ; 414).

8. Conducteur (110 ; 210 ; 310 ; 410) selon l'une quelconque des revendications précédentes, **caractérisé en ce qu'**on trouve un moyen d'ancrage (140 ; 228 ; 328 ; 428) au niveau de la partie proximale du câble conducteur (114 ; 214 ; 314 ; 414).

9. Conducteur (10 ; 110 ; 210 ; 310 ; 410) selon la revendication 6 ou 7, **caractérisé en ce qu'**il y a une section de câble conducteur libre (30 ; 130 ; 230 ; 330 ; 430) sans moyen d'ancrage entre le moyen d'ancrage (28 ; 128 ; 228 ; 328 ; 428) situé à proximité de la section d'électrode (16 ; 116 ; 216 ; 316 ; 416) et la section d'électrode (16 ; 116 ; 216 ; 316 ; 416).

10. Conducteur (10 ; 110 ; 210 ; 310 ; 410) selon la revendication 9, **caractérisé en ce que** la section de câble conducteur libre (30 ; 130 ; 230 ; 330 ; 430) a une longueur minimum de sélection dans une plage de 5 mm, particulièrement une longueur de sélection dans une plage de 10 mm à 60 mm et/ou que le moyen d'ancrage (28 ; 128 ; 228 ; 328 ; 428) a une longueur d'au moins 10 mm ou davantage, particulièrement de 60 mm ou davantage.

11. Conducteur (410) selon l'une quelconque des revendications précédentes, **caractérisé en ce que** le conducteur (410) comprend une boucle à relief de contrainte (440).

12. Conducteur (10 ; 110 ; 210 ; 310 ; 410) selon l'une quelconque des revendications précédentes, **caractérisé en ce que** le conducteur (10 ; 110 ; 210 ; 310 ; 410) est un conducteur de neurostimulation de moelle épinière, dans lequel la section d'électrode (16 ; 116 ; 216 ; 316 ; 416) est particulièrement une palette d'électrodes (18 ; 118 ; 218 ; 318 ; 418) avec une batterie d'électrodes (20 ; 120 ; 220 ; 320 ; 420), par exemple configurée pour être implantée à proximité de la section cervicale ou thoracique ou lombaire ou sacrée de la moelle épinière.

13. Conducteur (510 ; 610) selon l'une quelconque des revendications précédentes, **caractérisé en ce que** la section d'électrode (516 ; 616) du conducteur (510 ; 610) comprend au moins un moyen d'ancrage (528 ; 628) et/ou est directement reliée à au moins un moyen d'ancrage (528 ; 628) .

14. Conducteur (110) selon la revendication 1, **caractérisé en ce qu'**il comprend en outre un second moyen d'ancrage (140) séparé du moyen d'ancrage (128) sur le même câble conducteur (114).

15. Conducteur (110) selon la revendication 14, **caractérisé en ce que** le second moyen d'ancrage (140) est formé au niveau de la partie proximale du câble conducteur (114) .

16. Conducteur (210) selon la revendication 1, **caractérisé en ce que** le moyen d'ancrage (228) est configuré pour recouvrir plus de 2/3 de la longueur du câble conducteur (210).

17. Conducteur (310) selon la revendication 1, dans lequel le conducteur comprend une pluralité de câbles conducteurs (312, 314), **caractérisé en ce que** le moyen d'ancrage (328, 328a) est prévu à l'extrémité distale de chaque câble parmi ladite pluralité de câbles conducteurs (312, 314), dans lequel en option un second moyen d'ancrage est prévu à l'extrémité proximale de chaque câble parmi ladite pluralité de câbles conducteurs (312, 314).

18. Conducteur (410) selon la revendication 11, **caractérisé en ce que** la boucle à relief de contrainte (440) est recouverte par le moyen d'ancrage (428).
